Europäisches Patentamt

(19) European Patent Office    (11) Publication number: **0 1·13 129**

Office européen des brevets    **B1**

⑫    **· EUROPEAN PATENT SPECIFICATION ·**

(45) Date of publication of patent specification: **29.04.87**    (51) Int. Cl.⁴: **C 23 F 15/00, C 07 C 51/41,**
**C 07 C 53/126,**
(21) Application number: **83113211.3**    **C 10 M 129/38,**
(22) Date of filing: **29.12.83**    **C 10 M 101/02**

(54) Corrosion inhibitor for the protection of sheet metal.

(30) Priority: **31.12.82 PL 239943**
**31.12.82 PL 239942**

(43) Date of publication of application:
**11.07.84 Bulletin 84/28**

(45) Publication of the grant of the patent:
**29.04.87 Bulletin 87/18**

(84) Designated Contracting States:
**CH DE FR GB IT LI**

(56) References cited:
**GB-A-2 005 300**
**US-A-3 539 514**

**R.C.MEHROTRA, R.BOHRA, Metal
Carboxylates, pp. 19, 330 (Academic Press,
1983)**
**Kirk-Othmer, Encyclopedia of Chemical
Technology, vol. 14, p. 502**

(73) Proprietor: **Instytut Mechaniki Precyzyjnej**
**ul. Duchnicka 3**
**Warszawa (PL)**
(73) Proprietor: **Politechnika Gdanska**
**ul. Majakowskiego 11/12**
**Gdansk (PL)**

(72) Inventor: **Szauer, Tadeusz**
**ul. Worcella 2/4**
**Gdansk (PL)**
Inventor: **Kozlowski, Andrzej**
**Al. Konfederacji 68**
**Warzawa (PL)**
Inventor: **Iwanow, Jerzy**
**ul. Neseberska 1/12**
**Warszawa (PL)**
Inventor: **Brandt, Andrzej**
**ul. Poprzeczna 2**
**Starogard Gdanski (PL)**
Inventor: **Darowicki, Kazimierz**
**ul. Dickensa 2/8**
**Gdansk (PL)**
Inventor: **Wlodarczyk, Sylwia**
**ul. Schillera 4/3**
**Warszawa (PL)**

Courier Press, Leamington Spa, England.

**0 113 129**

74) Representative: **Hansen, Bernd, Dr.rer.nat. et al
Hoffmann, Eitle & Partner Patentanwälte
Arabellastrasse 4
D-8000 München 81 (DE)**

## Description

The present invention relates to corrosion inhibitors for protective oils and greases.

The inhibitor may be used in protective oils utilized for conservation of metal product surfaces after plastic or mechanical working, e.g. for conservation of motor-car body sheets stored in packs.

The inhibitor may also be used in protective greases and preservative gear greases.

British Patent Specification No. 1455250 discloses a corrosion inhibitor used in fuel tanks. This known inhibitor contains sodium nitrite, sodium benzoate and a small quantity of carboxyethylcellulose. The inhibitor is introduced into the fuel tanks in the form of tablets. This known inhibitor is usless as an inhibitor for protective oils.

From German Patent Specification No. 14 44 904, the addition of oils of benzotriazole additives along with maleic acid anhydride as corrosion inhibitors is known. Benzotriazole additives are effective working protectives for copper and its alloys, while they are less effective as concerns iron.

There are also known corrosion inhibitors added to protective oils and engine oils or to oils used as hydraulic fluids, which are benzotriazole compounds and oxazoline compounds containing an alkyl radical, alkene radical or cycloalkyl radical with a number of carbon atoms from 6 to 20. Oils containing small quantities of this known inhibitor show good anti-corrosive properties as engine oils and other working oils. They have insufficient protective properties as preservative oils for iron.

There are known barium derivatives of lanoline and barium sulphonate used as corrosion inhibitors for working greases. These known inhibitors show relatively small anti-corrosive efficiency and they are useless for protective oils, because after a short time of remaining on metal sheet surfaces or other metal surfaces, they are polymerized and they become hard to remove. This defect appears mainly in the conservation of sheets in packs. After protection with oil containing barium derivatives of lanoline and barium sulphonate as inhibitors, sheets in packs become agglutinated and are very hard to separate.

From the Polish Patent Specification No. 52975 and No. 48772, corrosion inhibitors are known which are derivatives of fatty acids, especially oleic acid amines.

From U.S. Patent Specification No. 4 166 151 corrosion inhibitors are known from oils and greases which are carboxylic acid esters with a carbon atom number $C_{10}$—$C_{25}$ and alcohol esters.

U..S. Patent Specification No. 4 168 292 discloses corrosion inhibitors for protective oils which are derivatives of fatty acids in form of acylic hydroxylaminealcylamides.

Polish Patent Application No. P 212732 discloses a composition for engine oil enriching, containing 1—50 parts by weight barium or calcium detergent with alkali reserve 60 mg KOH/g along with polymethylsilicon compound. The composition, among others, protects the metal parts of engines from various types of corrosion.

The fault of known inhibitors is a relatively small inhibiting efficiency regarding atmospheric corrosion. These means do not protect effectively against corrosion leading to the appearance of black corrosion spots, mainly during storage of sheets in packs. The known inhibitors are relatively expensive.

U.S. Patent Specification No. 4 212 751 and British Patent Specification No. 85 2365 disclose oils and bearing greases containing additives of such compounds as esters of dithiophosphoric acid, the salts of these esters and secondary alcohols in beta position having two quaternary carbon atoms. These compounds are known to improve the lubricity of oils which enables increase of the bearing loading and protects from pitting corrosion.

Polish Patent Application No. P 218353 discloses a grease oil containing 2—15% by weight of animal or vegetable sulphonated fats in the presence of a catalyst, 0.1—5% by weight of reaction product of fatty acids and/or naphthenic acids with polyalkylenopolyamine, 0.01—1% by weight of fatty amines or diamines and a composition of kerosenic hydrocarbons in an amount completed to 100% by weight.

U.S. Patent Specification No. 4 201 681 discloses a corrosion inhibitor for cooling fluids and for gear greases, being a mixture of barium derivative of lanoline and barium sulphonate.

The relevant defect of lubricant oils and greases containing the known inhibitors mentioned above is poor ability to mix permanently and homogeneously and to reach adequate stability of composition.

The defect of the known corrosion inhibiting agents is that any protection against corrosion is only temporary. The known inhibitors are hard to remove after a long period of storage and their anti-corrosive efficiency is relatively low.

The object of the invention is to provide a method for the protection of sheet metal using a protective oil containing a corrosion inhibitor for protective oils and greases free from the defects of known inhibitors, so that compositions based on this inhibitor will be less susceptible to the defects of compositions based on the known inhibitors.

The corrosion inhibitor for protective oils and greases based on alkali metal soaps used in the method according to the invention is characterised by containing vegetable and/or animal fatty acids with carbon chain length $C_{16}$—$C_{22}$, barium hydroxide, and a product of vegetable and/or animal fatty acids with chain length $C_{16}$—$C_{22}$ with barium hydroxide, the reaction proceeding so that first barium hydroxide is introduced into the fatty acids preheated to the temperature of about 120°C and this temperature is maintained during a predetermined period of time, and next the temperature of reactants is raised to about 160°C to 180°C and the reaction is maintained for a predetermined time, and the molar proportion of fatty acids to barium hydroxide amounts to from 1: 0.1 to 1: 2.

It is advisable that the corrosion inhibitor used in the method according to the invention contains also fractional amines $C_{12}$—$C_{22}$ and/or fractional amines $C_{12}$—$C_{22}$ and/or fatty alcohol esters of fraction $C_{12}$—$C_{22}$, with fatty acids of fraction $C_6$—$C_{22}$, and/or the salts of fatty amines of fraction $C_{12}$—$C_{22}$ and fatty acids of fraction $C_6$—$C_{22}$ and/or ethoxylic esters of fatty alcohols and ethoxylic fatty amines, preferably in an amount from 0.01 to 10% by weight.

It is desirable for the corrosion inhibitor used in the method according to the invention for protective oils and greases to contain a fatty acid reaction product with barium hydroxide; the reaction being conducted so that the temperature of the reactants preheated to about 120°C is maintained for about 2 h and the temperature of the reactants is about 180°C for 1 h.

It is advisable for the corrosion inhibitor used in the method according to the invention to contain the fatty acid reaction product with barium hydroxide; the reaction being conducted so that the temperature of the reactants preheated to about 120°C is maintained for about 1 h and the temperature of the reactants is about 180°C for about 2 h.

It is advantageous for the corrosion inhibitor used in the method according to the invention used for greases to contain also zinc hydroxide and a reaction product of a vegetable and/or an animal fatty acid having carbon chain length $C_{16}$—$C_{22}$ with zinc hydroxide, where the ratio of the amount of zinc hydroxide to barium hydroxide amounts to from 0.01:10 to 10: 0.01 by weight, and the ratio of the quantity of fatty acid to the sum of hydroxide quantity amounts in molar form to 1: 0.1 to 1:1.

It is advsisable if the corrosion inhibitor used in the method according to the invention used for greases contains also mineral oil in a quantity of from 1 to 50% by weight introduced into the reactive composition at a temperature of about 120°C, at least a few minutes before the temperature is raised to about 180°C.

It is desirable if the corrosion inhibitor used in the method according to the invention used for protective oils contains also orthophosphoric acid and/or 1- and/or 2- and/or tri-substituted organic derivatives of phosphoric acid in a quantity of from 0.01 to 5% by weight.

The inhibitors used in the method according to the invention used as additives even in very small quantities with known anti-corrosive agents, such as aliphatic amides, polyamines, polyalkanoloamines, heterocyclic amines, the salts of amines and fatty acids, amides, esters of ethyoxylic amines and fatty acids, improve functioning of the inhibitor much more than could be expected from the quantity of inhibitor added, taking into consideration the summing of inhibiting properties. This important improvement of inhibitor properties could result from cooperation of known compounds with the inhibitor according to the invention, which could be considered as a synergistic effect.

The surfaces coated with inhibitor used in the method according to the invention are resistant to emulsification and durable i.e. their protective properties do not deteriorate with time.

The corrosion inhibitor used in the method according to the invention is illustrated by the following manufacturing examples.

### Example I

Into the reactor are introduced 50 parts by weight of fatty acids with chain length $C_{16}$—$C_{22}$ which are heated to 120°C, slowly adding with constant mixing 60 parts by weight of barium hydroxide. Next the mixture is heated to the same temperature with constant mixing. The temperature is then raised to 160°C and with constant mixing it is maintained for 30 minutes. After cooling down the contents of the reactor to ambient temperature, the inhibitor may be introduced into protective oils.

### Example II

Corrosion inhibitors are made by mixing, at a temperature of 120°C, 2.5 parts by weight of inhibitor manufactured according to Example I and with 0.5 parts by weight of triethanol amine oleate. After cooling down the inhibitor made in such a way may be introduced into protective oils.

### Example III

To the corrosion inhibitor made as in Example I are added, at a temperature of 80°C, 0.7 parts by weight of orthophosphoric acid and the mixture is cooled down to ambient temperature. The inhibitor made in such a way may be introduced into protective oil.

Comparative researches of protective properties of inhibitors are carried out in accordance with the Polish Standard PN-72/H-04637. For examination plates of dimension 100 × 75 × 1 cut down from metal sheets coiled, roller and deep drawn were used. Before testing, the plates were degreased in extraction naphtha. The plates protected with the agents under test and the unprotected plates were placed in a "Stevenson Shed" for four months. Based on a determination of the amount of deterioration caused by corrosion, it was found that compared with the unprotected samples the power of inhibiting atmospheric corrosion is as follows:

— for samples protected with machine oil M8 — 30%

— for samples protected with machine oil M8 + 5% of commercial inhibitor, oleic acid amine — 50%

— for samples protected with machine oil M8 + 5% according to Example I — 80%

— for samples protected with machine oil M8 + 5% of inhibitor according to Example II — 95%

— for samples protected with machine oil M8 + 5% of inhibitor according to Example III — 95%

### Example IV

50 kg of fatty acid of fraction $C_6$—$C_{22}$ with acid number 150 and iodine value 15 is introduced into a reactor, the mixture is heated to 120°C and then 16 kg of a mixture of zinc hydroxide with barium hydroxide, containing 50% of zinc hydroxide is introduced. It is heated and the temperature of the mixture is maintained at a level of 120°C for 1 h. The mixture is next heated to 180°C and this temperature is maintained for 115 minutes. The mixture obtained in such a way is cooled down to ambient temperature. The extracted product is an effective inhibitor of atmospheric corrosion, very useful in grease production, especially for bearing lubricants.

### Example V

Into the inhibitor made according to e.g. Example I after 30 minutes of keeping the composition at a temperature of 120°C is added 25 kg of mineral fluid of low viscosity under the commercial name Selektol 10-W30 and the temperature is raised to 180°C. The temperature is maintained for 130 minutes. The content of the reactor is cooled down to ambient temperature. The inhibitor made in such a way is useful in production of semifluid polyfunctional greases with increased anti-corrosive properties, useful mainly for conservation of precision mechanisms.

### Example VI

Into the inhibitor made according to e.g. Example I after 45 minutes of maintaining the composition at a temperature of 120°C is added 1 part by weight of ethoxylic fatty alcohol esters with carbon chain length $C_{18}$—$C_{22}$ and containing 14 ethoxylic groups. The temperature is raised to 180°C and maintained for 15 minutes. Then it is cooled down to ambient temperature. The inhibitor made in such a way is useful for anticorrosive greases, especially for preservation of goods stored in conditions of increased humidity.

## Claims

1. A method for the protection of sheet metal by providing the sheets with a film of protective oil, characterized in that the said oil contains vegetable and/or animal fatty acids with carbon chain length $C_{16}$—$C_{22}$, in that it contains barium hydroxide, and in that it contains a reaction product of vegetable and/or animal fatty acids having carbon chain length $C_{16}$—$C_{22}$ with barium hydroxide, the reaction being conducted in that first barium hydroxide is introduced into a fatty acid preheated to a temperature of about 120°C, this temperature of reactants is maintained for a predetermined time and next the temperature of the reactants is raised to about 160°C to 180°C, and the reaction is maintained for the predetermined time, the molar ratio of fatty acids to barium hydroxide amounting to from 1: 0.1 to 1: 2.

2. A method for the protection of sheet metal by providing the sheets with a film of protective oil according to claim 1, characterized in that said oil also contains amines of friction $C_{12}$—$C_{22}$ and/or amides of fraction $C_{12}$—$C_{22}$ and/or fatty alcohol esters of fraction $C_{12}$—$C_{22}$ with fatty acids of fraction $C_6$—$C_{22}$ and/or the salts of fatty amines of fraction $C_{12}$—$C_{22}$ and of fatty acids of fraction $C_6$—$C_{22}$ and/or ethoxylic esters of fatty alcohols and ethoxylic fatty amines, preferably in a quantity of from 0.01 to 10% by weight of barium soap.

3. A method according to claim 1, characterized in that the reaction is conducted so that the temperature of the reactants preheated to about 120°C is maintained for about 2 h and the temperature of the reactants is maintained at about 180°C for 1 h.

4. A method according to claim 1, characterized in that the reaction is conducted so that the temperature of the reactants preheated to about 120°C is maintained for about 1 h, and the temperature of the reactants is maintained at about 180°C for about 2 h.

5. A method according to claim 1, characterized in that said oil also contains zinc hydroxide and a reaction product of vegetable and/or animal fatty acids with chain length $C_{16}$—$C_{22}$ with zinc hydroxide, and the ratio of quantity of zinc hydroxide to barium hydroxide amounts to from 0.1:10 to 10 : 0.01, and the ratio of fatty acid quantities to the sum of hydroxide quantity amounts in molar form to from 1: 0.1 to 1:1.

6. A method according to claim 1, characterized in that said oil also contains mineral oil in a quantity from 1 to 50% by weight introduced into the reaction mixture at a temperature of about 120°C at least a few minutes before raising the temperature to about 180°C.

7. A method according to claim 1, characterized in that said oil also contains orthophosphoric acid and/or 1- and/or 2- and/or 3-substituted organic derivatives of phosphoric acid in a quantity of from 0.01 to 5% by weight of barium soap.

## Patentansprüche

1. Verfahren zum Schutz von Stahlblech, in dem die Bleche mit einem Film aus Schutzöl versehen werden, dadurch gekennzeichnet, dass das Öl pflanzliche und/oder tierische Fettsäuren mit einer Kohlenstoffkettenlänge von $C_{16}$—$C_{22}$, Bariumhydroxid und ein Umsetzungsprodukt aus pflanzlichen und/oder tierischen Fettsäuren mit Kohlenstoffkettenlängen von $C_{16}$—$C_{22}$ mit Bariumhydroxid enthält, wobei die Umsetzung erfolgt indem zuerst Bariumhydroxid der Fettsäure zugegeben wird, die aus eine Temperatur von ca. 120°C vorerwärmt ist, diese Temperatur der Reaktionspartner für eine gegebene Zeit beibehalten und daraufhin die Temperatur der Reaktionspartner auf ca. 160°C bis 180°C erhöht und die Umsetzung für eine gegebene Zeit fortgeführt wird, wobei das molare Verhältnis der Fettsäuren zu Bariumhydroxid im Bereich von 1: 0,1 bis 1: 2 liegt.

2. Verfahren zum Schutz von Stahlblech nach Anspruch 1, in dem die Bleche mit einem Film aus Schutzöl versehen werden, dadurch gekennzeichnet, dass das genannte Öl auch Amine der Fraktion $C_{12}$—$C_{22}$ und/oder Amide der Fraktion $C_{12}$—$C_{22}$ und/oder Fettalkoholester der Fraktion $C_{12}$—$C_{22}$ mit Fettsäuren der Fraktion $C_6$—$C_{22}$ und/oder Salze von Fettaminen der Fraktion $C_{12}$—$C_{22}$ und von Fettsäuren der Fraktion $C_6$—$C_{22}$ und/oder Ethoxyester von Fettalkoholen und Ethoxylfettamine, vorzugsweise in einer Menge von 0,01 bis 10 gew.-% der Bariumseife enthält.

3. Verfahren nach Anspruch 1, dadurch gekennzeichnet, dass die Umsetzung so durchgeführt wird, dass die Temperatur der Reaktionspartner, die auf 120°C vorerwärmt sind, ca. 2 h gehalten und die Temperatur der Reaktionspartner 1 h auf ca. 180°C gehalten wird.

4. Verfahren nach Anspruch 1, dadurch gekennzeichnet, dass die Umsetzung so durchgeführt wird, dass die Temperatur der Reaktionspartner, die auf 120°C vorerwärmt sind, ca. 1 h gehalten, und die Temperatur der Reaktionspartner 2 h auf 180°C gehalten wird.

5. Verfahren nach Anspruch 1, dadurch gekennzeichnet, dass das genannte Öl auch Zinkhydroxid und ein Umsetzungsprodukt aus pflanzlichen und/oder tierischen Fettsäuren mit Kettenlängen von $C_{16}$—$C_{22}$ mit Zinkhydroxid enthält, und das Verhältnis der Menge von Zinkhydroxid zu Bariumhydroxid im Bereich von 0,1:10 bis 10 : 0,01 liegt, und das Verhältnis der Fettsäuremengen zur Summe der Hydroxidmenge in molarer Form im Bereich von 1: 0,1 bis 1:1 liegt.

6. Verfahren nach Anspruch 1, dadurch gekennzeichnet, dass das genannte Öl auch Mineralöl in einer Menge von 1 bis 50 Gew.-% enthält, welches dem Reaktionsgemisch bei einer Temperatur von ca. 120°C zumindest einige Minuten vor Erhöhung der Temperatur auf ca. 180°C zugegeben wird.

7. Verfahren nach Anspruch 1, dadurch gekennzeichnet, dass das genannte Öl auch Orthophosphorsäure und/oder 1- und/oder 2- und/oder 3-substituierte organische Derivate von Phosphorsäure in einer Menge von 0,01 bis 5 Gew.-% der Bariumseife enthält.

**Revendications**

1. Un procédé pour la protection de métal de tôles par application d'un film d'huile protectrice sur les tôles, caractérisé en ce que ladite huile contient des acides gras d'origine végétale et/ou d'origine animale en $C_{16}$—$C_{22}$, en ce qu'elle contient de l'hydroxyde de barum et en ce qu'elle contient un produit de réaction d'acides gras d'origine végétale et/ou d'origine animale en $C_{16}$—$C_{22}$ avec l'hydroxyde de baryum, la réaction étant effectuée en introduisant d'abord l'hydroxyde de baryum dans un acide gras préchauffé à une température d'environ 120°C, en maintenant cette température des réactifs pendant une durée prédéterminée et ensuite en élevant la température des réactifs à environ 160—180°C et an maintenant la réaction pendant une durée prédéterminée, le rapport molaire des acides gras à l'hydroxyde de baryum étant de 1: 0,1 à 1: 2.

2. Un procédé pour la protection de métal en tôles par application sur les tôles d'un film d'huile protectrice selon la revendication 1, caractérisé en ce que ladite huile contient également des amines d'une fraction en $C_{12}$—$C_{22}$ et/ou des amides d'une fraction en $C_{12}$—$C_{22}$ et/ou des esters d'alcools gras d'une fraction en $C_{12}$—$C_{22}$ avec des acides gras d'une fraction en $C_6$—$C_{22}$ et/ou les sels d'amines grasses d'une fraction en $C_{12}$—$C_{22}$ et d'acides gras d'une fraction en $C_6$—$C_{22}$ et/ou des esters éthoxyliques d'alcools gras et des amines grasses éthoxyliques, de préférence en quantité de 0,01 à 10 % en poids par rapport au savon de baryum.

3. Un procédé selon la revendication 1, caractérisé en ce que la réaction est effectuée en maintenant pendant environ 2 h la température des réactifs préchauffés à environ 120°C et en maintenant la température des réactifs à environ 180°C pendant 1 h.

4. Un procédé selon la revendication 1, caractérisé en ce que la réaction est effectuée en maintenant pendant environ 1 h la température des réactifs préchauffés à environ 120°C et en maintenant la température des réactifs à environ 180°C pendant environ 2 h.

5. Un procédé selon la revendication 1, caractérisé en ce que ladite huile contient également de l'hydroxyde de zinc et un produit de réaction d'acides gras d'origine végétale et/ou d'origine animale en $C_{16}$—$C_{22}$ avec l'hydroxyde de zinc et le rapport de l'hydroxyde de zinc à l'hydroxyde de baryum est de 0,1:10 à 10 : 0,1 et le rapport molaire des acides gras à la somme des hydroxydes est de 1:0,1 à 1:1.

6. Un procédé selon la revendication 1, caractérisé en ce que ladite huile contient également une huile minérale en quantité de 1 à 50 % en poids introduite dans le mélange de réaction à une température d'environ 120°C au moins quelques minutes avant d'élever la température à environ 180°C.

7. Un procédé selon la revendication 1, caractérisé en ce que ladite huile contient également de l'acide orthophosphorique ou des dérivés organiques mono- et/ou di- et/ou tri-substitués de l'acide phosphorique en quantité de 0,01 à 5 % en poids du savon de baryum.